# EUROPEAN PATENT SPECIFICATION

(11) **EP 0 774 920 B1**
(45) Date of publication and mention of the grant of the patent: **10.09.2003**
(21) Application number: 95926582.8
(22) Date of filing: 26.07.1995
(51) Int. Cl.: A61B 6/04, A61B 6/00, H05G 1/02

(54) **A PATIENT TABLE HAVING A RECEPTOR UNIT FOR RADIATION SENSING, SUCH AS X-RAY PHOTOGRAPHY OR ELECTRONIC IMAGE STORAGE**
PATIENTENTISCH MIT EINER EMPFANGSEINHEIT FÜR STRAHLUNGSSENSOREN WIE RÖNTGENAUFNAHMEVORRICHTUNGEN ODER ELEKTRONISCHE BILDSPEICHERMEDIEN
TABLE DE SUPPORT DE PATIENT DOTEE D'UN MODULE RECEPTEUR DES RAYONS TEL QUE RADIOGRAPHIE OU ENREGISTREMENT ELECTRONIQUE D'IMAGES

(30) Priority: 28.07.1994 SE 9402589
(43) Date of publication of application: 28.05.1997
(73) Proprietor: HOLOGIC, INC., Bedford, Massachusetts 01730-1401 (US)
(72) Inventor: OHLSON, Carl-Eric, S-115 24 Stockholm (SE)
(74) Representative: Wennborg, Göte
(86) International application number: SE9500887
(87) International publication number: WO96003077

(56) References cited:
- SE-B- 463 237
- US-A- 4 468 803
- US-A- 5 157 707
- Philips "Diagnost 76" brochure

## Description

### TECHNICAL FIELD

In imaging processes, there is used a radiation source which is supported for movement in X-, Y- and Z-directions and which is rotatable about a horizontal axis. A receptor unit may be mounted in or positioned beneath a patient table and is movable in the X-direction. Movement of the radiation source may be initiated automatically, as the receptor unit is moved.

By X-direction is meant here and in the following a direction of movement which is parallel with one long side of the patient table, while by Y-direction is meant a direction of movement perpendicular to the extension of said long side, i.e. a direction of movement parallel with the short sides of the table. By Z-direction is meant movement in a vertical direction. This enables the patient table to be brought to different positions in relation to a tower column or a ceiling-mounted tower which carries the beam source.

The present invention relates to a patient table equipped with a receptor unit, and more specifically to a patient table of the kind defined in the preamble of Claim 1.

### BACKGROUND PRIOR ART

GB-B-1,323,769 (Picker Corp.) describes apparatus comprising a receptor part disposed in a patient support table, and an overlying ceiling-mounted beam source. The apparatus enables side-on photographs to be taken with a horizontal beam path, by swinging-up the patient's support table about a horizontal axis and pivoting the beam source. The apparatus also enables the image size and the shutter setting to be varied in relation to the beam-source/receptor distance ("SID", i.e. "source-image-distance". However, movement of the beam source and swinging of the patient support table must be effected manually, which is experienced as troublesome by the radiologists concerned.

EP-A-0 430 934 (AO Medical Products) describes a patient table of the aforesaid kind in which activation of a secondary receptor pivotally associated with the receptor unit or mountable thereon and extending in a vertical plane results, optionally after a time delay, in automatic movement of the beam source to a basic setting for horizontal, centered beam path onto the secondary receptor.

A Philips "Diagnost 76" brochure describes a patient support table which carries a receptor unit for a vertical beam path. This receptor unit can be swung outwards and upwards from one side of the table, to a position for receiving a horizontal beam path.

This latter arrangement which is considered to represent the nearest prior art, and comprises the features stated in the preambe of claim 1, has a number of drawbacks. When the receptor unit is to be swung out and up to receive a horizontal beam path, it is necessary for personnel who need to stand on the other side of the table in order to manoeuver the beam source to move around the table to swing the receptor unit outwards and upwards, and then move back around the table and place themselves in their original position in which the beam source can be manoeuvered. This procedure is experienced by the personnel as being both troublesome and time-consuming. The described solution also has other drawbacks from an ergometric aspect.

Another drawback is that the receptor unit is not centred in relation to the beam path when swung outwards and upwards from the table, and it is therefore necessary to move the beam source in the X-direction when adjusting for horizontal beam path.

Furthermore, this known arrangement only allows an exposure to be taken from one side of the patient. It is often difficult to "turn" the patient, particularly when the patient is seriously injured.

The effect of these drawbacks may sometimes be so serious as to impair the clarity of the pictures to an extent such as to require the X-ray to be taken again, therewith exposing the patient to an unnecessarily high radiation dosage.

### THE OBJECTS OF THE INVENTION

One object of the invention is to provide a patient support table of the aforedefined kind which, with one and the same receptor equipment, enables pictures to be taken with a vertical beam path, for instance with the patient lying down, and also with a horizontal beam path from each side of the patient support table, and preferably without changing the setting of the beam source in the X-direction, i.e. without moving the beam source laterally.

Another object is to provide equipment of the aforesaid kind which is superior to earlier known equipment with regard to ergometrics.

A further object of the invention is to provide equipment which can be adapted readily to different specific parameters, such as receptor size, table width, etc.

### BRIEF DESCRIPTION OF THE INVENTION

These and other objects are fulfilled with a patient table of the kind defined in the preamble of Claim 1 and having the features set forth in the characterizing clause of said Claim.

The invention enables the beam to be kept central in the Y-direction in the transition between a vertical and a horizontal beam path, irrespective of the alignment of the horizontal beam path in the Y-direction. In addition, the invention enables a receptor that has been swung up to a position above the table to be moved towards the patient and therewith obtain optimum picture sharpness and therewith a clearer diagnosis from the picture or image obtained.

The invention also enables the positions of pivot centres to be determined accurately in accordance with the different parameters that apply in individual cases, for instance in accordance with the dimensions of the receptor unit, the width of the patient support table, the desire for the bottom edge of the receptor to be located at a given height above the table when the receptor is in an outwardly swung position, and so on.

The present invention also enables the introduction of mutually dependent locking facilities with a crosswise function for outward swinging of the receptor unit in the horizontal plane. The effect afforded by the invention can be likened to the hinge of a hinged door, i.e. the receptor can present alternative pivot centres in dependence on the direction in which the receptor unit is swung outwards. Left and right pivot centres can be readily adapted to occurrent types of tables. In practice, it is preferred that respective pivot centres are so placed in the X- and Y-directions that centering of the beam source in the X-direction will be the same for both a horizontal and a vertical beam path. A preferred table according to the invention permits swinging the receptor unit in one or both of the alternative outwardly and upwardly swung positions to a position in which the unit is perpendicular to the longitudinal axis of the table, therewith enabling X-ray pictures to be taken of a patient seated in a wheelchair, for instance.

This enables the requirement of a separate frame or stand for taking such pictures to be dispensed with. This special outwardly swung position of the receptor unit may also be used in other circumstances, for instance when taking lung X-rays, etc.

In one preferred table according to the invention, the receptor unit is movable in the X-direction along the longitudinal axis of the table with corresponding automatic movement of the beam source and resetting of said source for a horizontal beam path towards the receptor unit, after having swung the receptor unit outwardly and upwardly beyond a side edge of the table.

Thus, in the case of this preferred table, the beam source is adjusted automatically to the position adopted by the receptor unit.

In another preferred table, the receptor unit is supported by a carriage which can move in the X-direction relative to the table and which can also be moved in the Y-direction relative to the carriage. The unit is carried by arms whose lengths can be adjusted and which supports the unit stably and reliably.

The receptor unit can then be dropped down or raised up from an outwardly swung horizontal position on each side of the table, to a respective vertical position beneath or above the table, by pivotal movement about mutually parallel axles located at different levels.

When in an outwardly swung and upwardly lifted vertical position, the receptor unit may further be swung about a central, vertical axle for work with an angled beam path.

### BRIEF DESCRIPTION OF THE DRAWINGS

The invention will now be described in more detail with reference to exemplifying embodiments thereof and also with reference to the accompanying drawings, in which
Fig. 1 is a perspective view of a receptor unit which can be swung outwardly and upwardly in different directions in accordance with the invention and which is mounted on a patient support table shown in chain lines;
Fig. 2 is a perspective view of the patient support table shown in Fig. 1 with the receptor unit in an outwardly and upwardly swung position, referenced C, to the right of the patient table, wherein the Figure also shows a number of possible alternative positions of the receptor unit, referenced B, D, E, F and G respectively, wherein the initial position shown in Fig. 1 is referenced A;
Figs. 3-9 show the patient support table from above with respective receptor units in the aforesaid different positions, wherein Fig. 3 corresponds to positions A, Fig. 4 corresponds to position B, Fig. 5 corresponds to position C, Fig. 6 corresponds to position D, Fig. 7 corresponds to position E, Fig. 8 corresponds to position F and Fig. 9 corresponds to position G;
Fig. 10 is a view of the patient support table shown in the other Figures from above, with the receptor unit shown in positions A and B, wherein the Figure also shows a number of reference signs relating to different relevant measurements and distances regarding the patient support table and the receptor unit respectively as explained in more detail below, and wherein the figure thus illustrates the geometric relationship between occurrent magnitudes;
Fig. 11 is a side view illustrating different receptor positions;
Fig. 12 is a simplified principle perspective view of a modified design of the patient support table, in which the receptor unit is accommodated in a carriage which can be moved along the table in the X-direction and is so mounted in the carriage as to be also movable in the Y-direction;
Fig. 13 is a perspective exploded view illustrating some of the elements by means of which the receptor unit is supported by the carriage for movement in the Y-direction;
Fig. 14 is a perspective view of the table and the carriage-supported receptor unit in a position in which the unit lies partially outside the table, i.e. prior to swinging the receptor unit outwards;
Fig. 15 is a perspective view corresponding to the view of Fig. 14 and shows the receptor unit swung out away from the table;
Fig. 16 is a perspective view corresponding to the view of Fig. 15 but showing the receptor unit swung to a vertical position;
Fig. 17 is a perspective view of the arrangement shown in Figs. 12-16, wherein the receptor unit has been swung down to a vertical position about an axle which is parallel with the axle used to swing-up the unit, this downwardly swung position being used, for instance, to take X-rays of the knees of a seated patient;
Fig. 18 is a perspective view of parts of those elements which function in the linear and pivotal movements of the receptor unit;
Fig. 19 is a perspective view showing that the receptor unit can be swung about a vertical axle relative to its carrying means, so as to enable pictures to be taken with an angled beam path; and
Fig. 20 is a perspective principle view illustrating some of those positions to which the receptor unit can be adjusted by means of the carrying and journalling arrangement shown in Figs. 12-19.

### DESCRIPTION OF PREFERRED EMBODIMENTS

The reference numeral 1 used in the various Figures identifies a patient support table for use in X-ray photography or in some other type of beam sensing, such as electronic image storage, for instance. The patient support table is supported on telescopic legs 1a provided with floor plates 1b, which enable the table to be adjusted vertically.

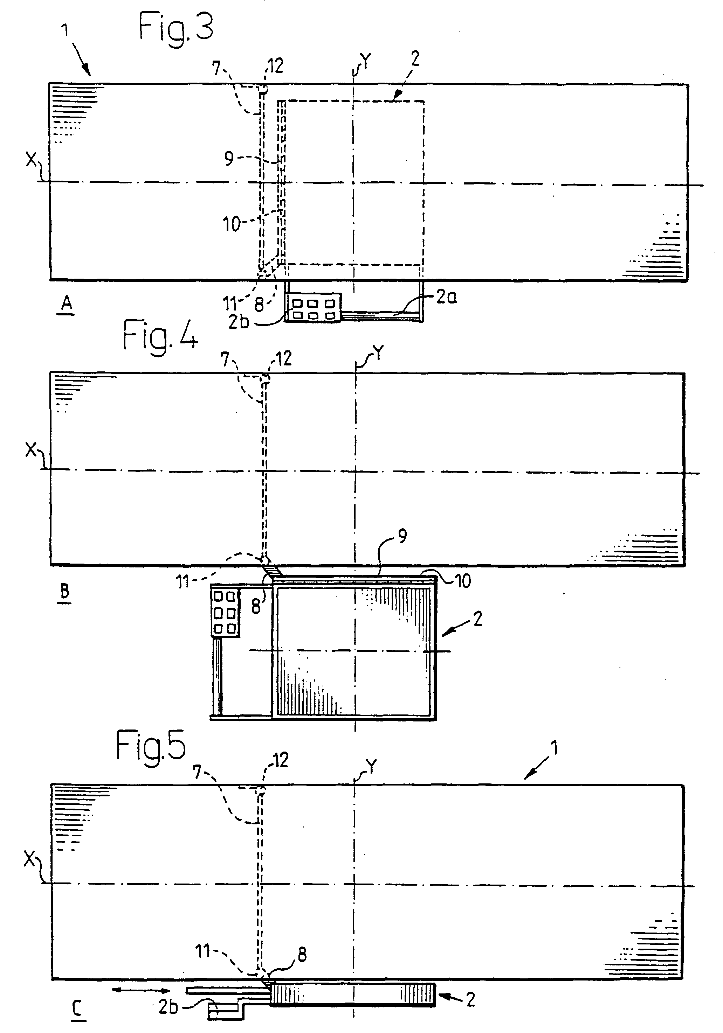

The table includes a recess or aperture 5 for the accommodation of a receptor unit, generally referenced 2. The receptor unit can be moved in the X-direction, i.e. in the longitudinal direction of the table. Some of the Figures 3-9 show an X-axle in the centre of the table. To this end, the receptor unit may be mounted on a carriage or like device (not shown) mounted in the table.

The receptor unit 2 is intended for coaction with a beam source (not shown) which can be moved in the X-direction, the Y-direction, i.e. transversely to the long axis of the table, and in the Z-direction, i.e. in a vertical direction. The beam source can also be swung about a horizontal axle.

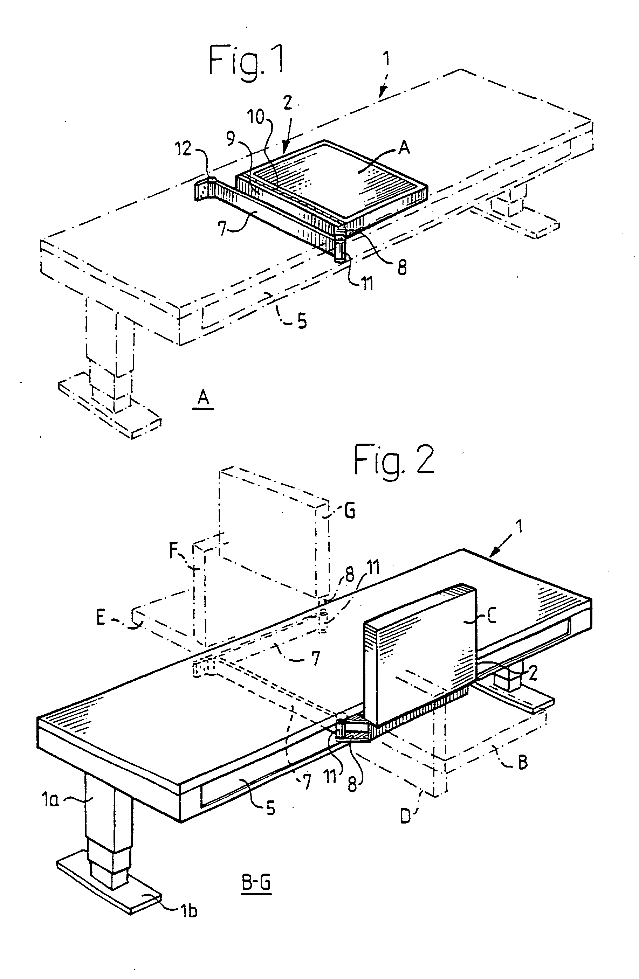

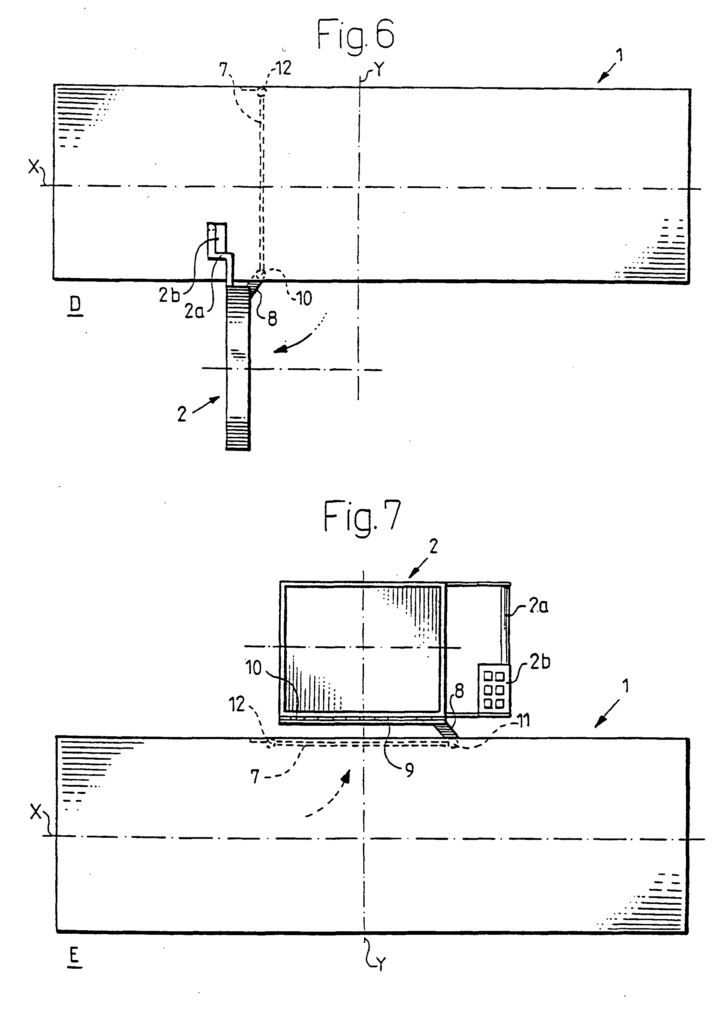

The receptor unit 2 is supported by arms 7, 9 which are joined together via a link 8 and which coact with pivot centres 11, 12 having vertical pivot axles located in the region of each side edge of the table, such as to enable the receptor unit to be swung out to alternative positions on each side of the table. The arm 9 is connected to the receptor unit 2 by means of a horizontal hinge 10. The receptor unit 2 can thus be swung out from the initial position A shown in Fig. 1 to the position B shown in Fig. 2, this latter position also being shown in Fig. 4.

The receptor unit shown in Figs. 3-9 correspond to the receptor unit shown in Figs. 1 and 2, with the exception that the units shown in Figs. 3-9 have a handgrip 2a which enables the receptor unit to be swung manually. The receptor units are also provided with an operating panel 2b having push buttons by means of which different receptor locking and receptor release operations can be initiated, the beam source activated, etc.

The receptor can be swung up about the horizontal hinge 9 from the position B shown in Fig. 4 to the position C shown in full lines in Figs. 2 and 5.

The vertical axle 11 enables the receptor unit to be swung from position C to a position D, shown in Fig. 6, in which the receptor unit is at right angles to the table 1. With the receptor unit in position D, side-on pictures and front-on pictures can be taken of a patient seated in a wheelchair, for instance.

In Fig. 7, the receptor unit 2 has been swung from the initial position shown in Figs. 1 and 3 in the other direction, about the vertical axle 12, to the position E in which the receptor unit is located slightly outside the opposing side edge of the table. It will be seen that in this position the receptor unit is also centered in the X-direction, i.e. there is no need to move the beam source laterally.

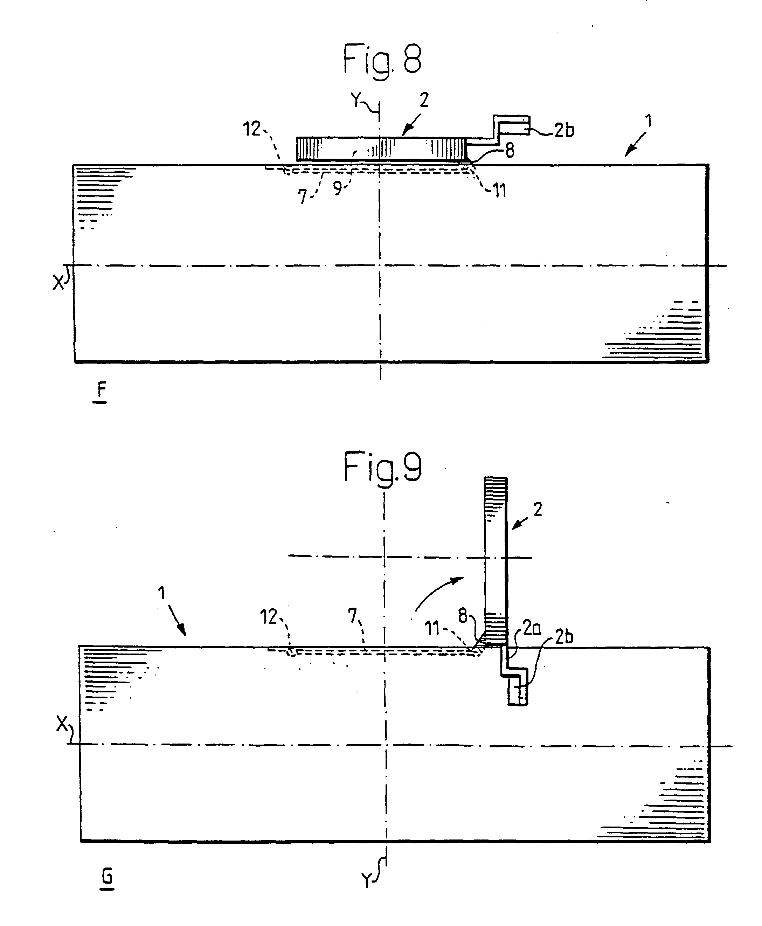

The receptor unit can be swung up from the position E shown in Fig. 7 to the position F shown in Fig. 8.

Fig. 9 illustrates the receptor unit swung from position F to position G, this position corresponding to the position D on the other side of the table.

The various Figures illustrate that when applying the inventive method, the beam can be kept centered in the X-direction in the transition between the vertical beam direction and the two horizontal beam directions, in accordance with the position shown in Figs. 1 and 2 and in Figs. 7 and 8 respectively. At the same time, the positions of the two pivot centres 11 and 12 can be determined as desired, in a manner described in more detail below.

The pivot axle about which the receptor unit is swung up is placed so that when the unit is in an upwardly swung position, the bottom edge of the unit will be located roughly in the plane of the table top or above the table top, for instance at a distance of 20 mm therefrom.

One advantage is that the operating unit 2a, 2b is located on the same side of the table as the radiologist or his/her assistant, therewith facilitating operation.

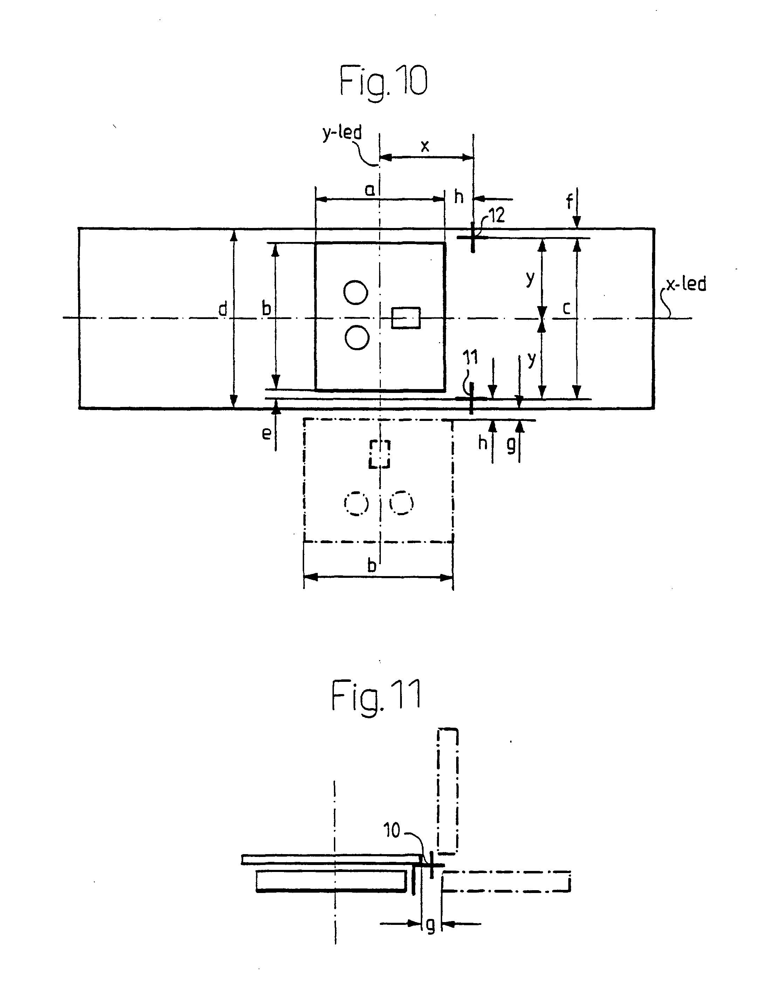

Figs. 10 and 11 illustrate different conceivable component measurements and the distances therebetween. The measurements also correspond to a left-hung receptor unit. In the initial position of the receptor unit
a) is the width of the receptor unit in the X-direction;
b) is the length of the unit in the Y-direction;
c) is the distance between the two pivot centres 11 and 12;
d) is the width of the table;
e) is the distance in the Y-direction between the receptor unit and the pivot centre 11;
f) is the distance between one side edge of the table and the pivot centre 12;
g) is the distance in the Y-direction between the opposite side edge of the table and one end edge of the receptor unit in position B;
h) is the distance between this last-mentioned side edge and the pivot centre 11;
x) is the distance in the X-direction between the centre point of the receptor unit in position A and the pivot centres 11, 12;
y) is the distance in the Y-direction between the centre point of the receptor unit in position A and the pivot centres 11, 12; and
h) is a radius corresponding to the length of the link 8.

The following relationships will thus apply:
x = Y = z/2
e = (c - b)/2
f = (d - c)/2
g = (2 x c - a - d)/2
h = (c - a)/2

Table 1 below lists measurements which can be applied and calculated with regard to a constructional design preferred in practice.

**Table 1**

| Receptor width | Receptor depth | Variable Axle distance | Table width | Distance centre Receptor | Variable Centre Receptor | Receptor Plate | Centre Receptor |
|---|---|---|---|---|---|---|---|
| a | b | c | d | e | f | g | h |
| 479 | 580 | 590 | 700 | 20 | 55 | 1 | 56 |
| 564 | 580 | 590 | 700 | 20 | 55 | -42 | 13 |
| 479 | 580 | 600 | 700 | 40 | 50 | 11 | 61 |
| 564 | 580 | 600 | 700 | 40 | 50 | -32 | 18 |
| 479 | 580 | 610 | 700 | 60 | 45 | 21 | 66 |
| 564 | 580 | 610 | 700 | 60 | 45 | -22 | 23 |

The Table shows the aforesaid relationships in one application example, in which c and f are variables.

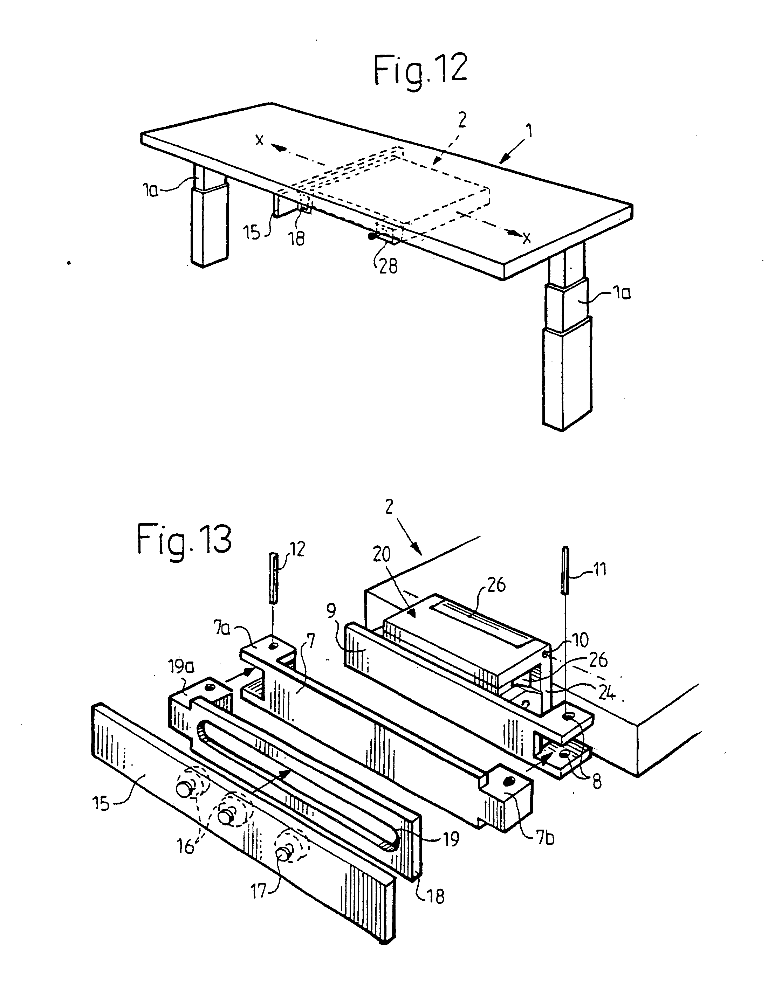

Figs. 12-19 show a modified design of the receptor unit 2, to-wit a design in which the receptor unit is carried for movement in the Y-direction by an element 15 which may either be part of a carriage which can be moved in the X-direction in relation to the Table 1 or form part of a stand or frame that is fixed in relation to the table.

In the case of the illustrated embodiment, the element 15 includes three rollers 16 which are carried for rotation on three horizontal axles 17 and which are disposed in an elongated slot 19 provided in a further element 18 and functioning to guide movement of the further element 18 in relation to the first-mentioned element 15.

The further element 19 is provided at one end with a bearing block 19a which coacts with a corresponding bearing block 7a on a corresponding end of the arm 7. The arm 7 of this embodiment is thus journalled more stably about the axle 12 than in the aforedescribed embodiments.

The other end of the arm 7 includes a bearing block 7b which coacts with a bearing element 8 corresponding to the bearing element 7a on the arm 7, this arrangement corresponding functionally to the link element 8 of the aforedescribed embodiments. The journal axle of this embodiment is referenced 11, as in the former cases.

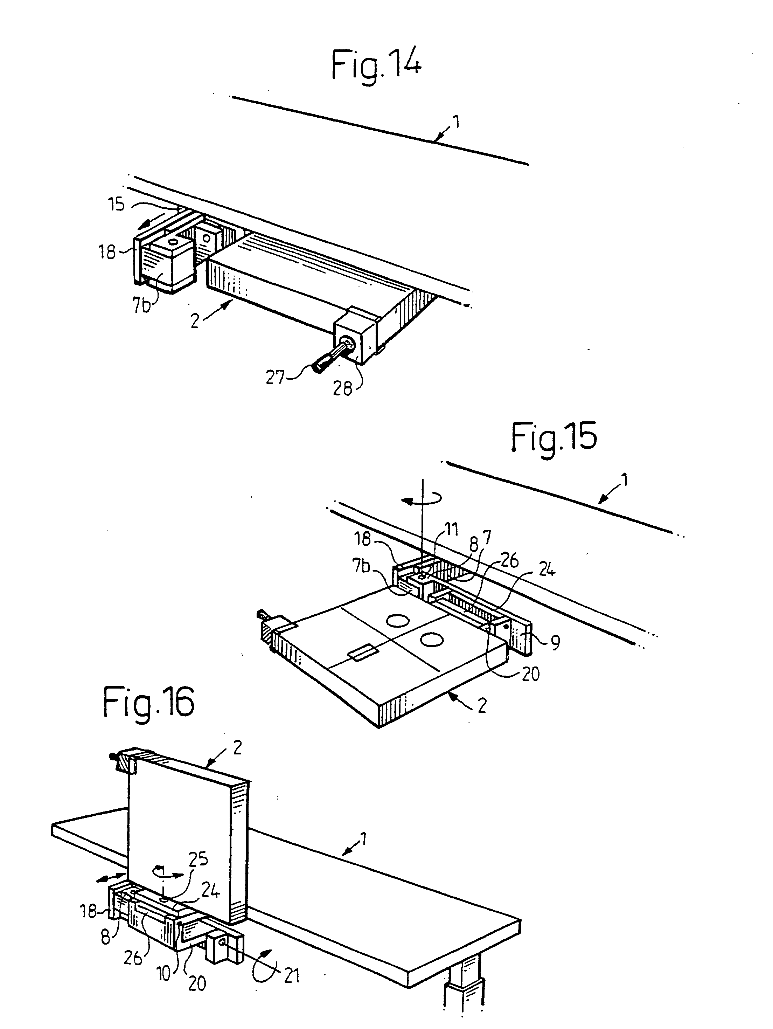

For the purpose of supporting the receptor unit 2, the arm 9 is firmly secured with the aid of an intermediate element 6 which is embraced by a U-shaped block 20 which is connected to the intermediate element 26 in a manner to allow the block 20 to pivot about the horizontal axle 10. As will be seen from Figs. 14-16, the receptor unit carried by the carrier and guide arrangement 15-19 can be displaced in the Y-direction relative to the element 15 to the position shown in Fig. 15 in which the receptor unit 2 lies outside the confines of the Table 1. The receptor unit can then be swung up to the vertical position shown in Fig. 16 about the axle 10.

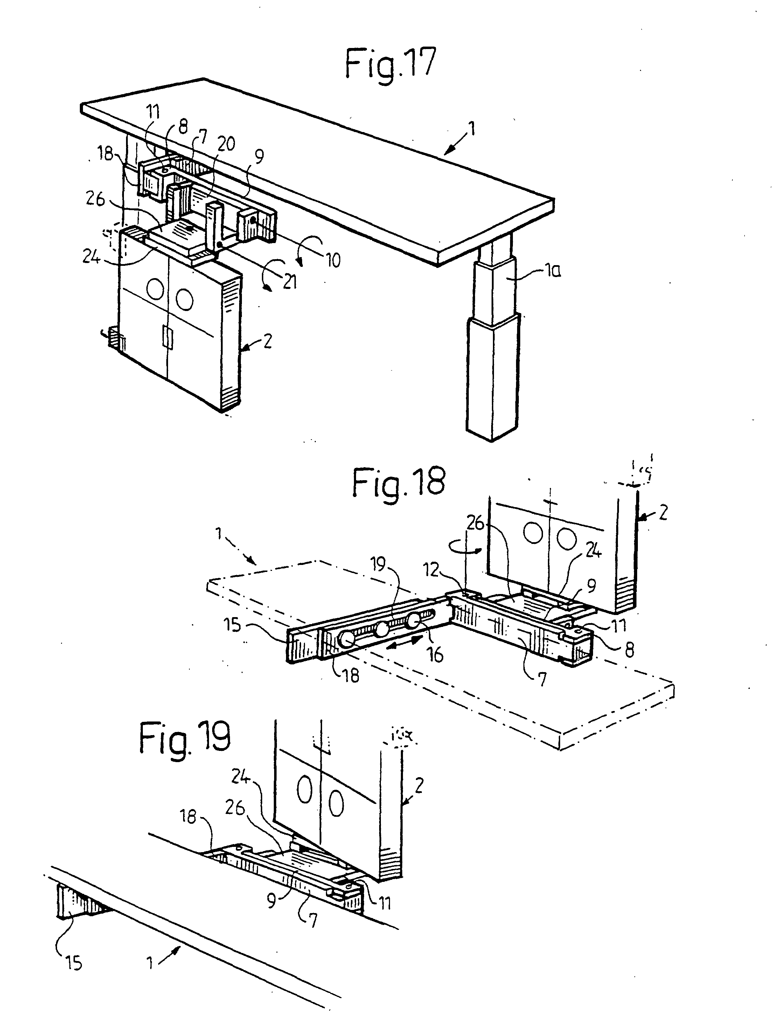

In Fig. 17, the receptor unit 2 is shown to be swung down to a vertical position which defines an angle of 180° with the position shown in Fig. 16. X-rays can be taken of the knees of a standing or sitting patient with the receptor unit in this position.

The receptor unit 2 is swung down around the axle 21, which is parallel with the axle 10 but located on a lower level than said axle.

The intermediate element 26 supports the receptor unit 2 through the medium of a plate 24. As will be seen from Fig. 16, the plate 24 is pivotal about a vertical axle 25. The receptor unit 2 accompanies the movement of plate 24 as it swings around the vertical axle 25, therewith enabling the receptor unit to be moved to the position shown in Fig. 19, for instance. In this position, the receptor unit is able to take pictures with an angled beam path.

Fig. 18 shows the elements 15-19 in an operative position, after having moved the receptor unit 2 in the Y-direction to the other side of the table 1 relative to the position shown in Fig. 17, and after having swung the receptor unit 2 first to a horizontal position about the axle 21, and thereafter to an upwardly swung, vertical position about the axle 10.

When the receptor-unit support element 15 forms part of a carriage which can be moved in the X-direction, the receptor unit can be moved in the X-direction from the position shown in Fig. 18. The beam source will normally accompany this movement of the receptor unit automatically. Movement of the unit in the X-direction and in the Y-direction can be achieved with the aid of appropriate motors (not shown).

As indicated in Figs. 14-19, the receptor unit 2 may include a unit 28 which carries a joy-stick 27 and which functions to facilitate movement of the receptor unit in different directions. Obviously, the unit 28 may be placed in some other position, for instance on one or both sides of the table or on a table-carried carriage (not shown).

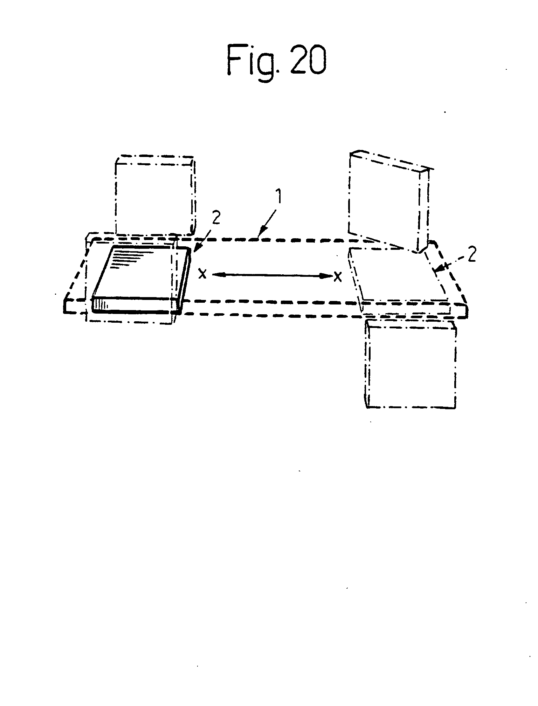

Fig. 20 is a perspective view illustrating principly the different positions to which a receptor unit 2 can be moved in relation to a patient support table 1 when using the carrier and bearing mechanisms illustrated in Figs. 12-19. Some of the positions shown by way of example in Fig. 20 correspond to the positions referenced A, C, F in Figs. 1 and 2. However, Fig. 20 shows a number of further positions which have been made possible because the receptor unit can be moved in the X-direction and also possibly in the Y-direction, and because the receptor unit can also be swung down from a horizontal position and adjusted about a vertical axle for operating with an angle beam path.

Other modifications of the invention are possible within the scope of its basic concept as expressed in the following Claims. For instance, the receptor unit may be accommodated in a frame which carries a table top, for instance a "floating" table top, i.e. a table top that is movable in the X-direction and/or the Y-direction.

The trend towards the development of filmless systems in which images are produced and stored electronically is particularly well served by the inventive patient support table. Because of the complexity of such electronic systems and because of the cost of such systems in which the receptor is connected directly to an evaluating unit, it is of extreme importance that the receptor can be used universally, therewith avoiding loose film cassettes, for instance.

## Claims

1. A patient support table equipped with a receptor unit and intended for radiation sensing such as X-ray photography or electronic image storage, wherein the receptor unit (2)
is supported for movement in an X-direction parallel to the longitudinal direction of the table (1);
adapted for coaction with a beam source which is movable in the X-direction, a Y-direction parallel to the transverse direction of the table and in a Z-direction;
adapted to be swung about a horizontal axle; and
capable of being swung out and up about a vertical and a horizontal axle from a position in or beneath a top surface of the table (1) in the case of a vertical beam path, to a position on one side of and parallel with the table in case of a horizontal beam path,
**characterized in**
**that** the receptor unit (2) is supported by an articulated structure (7-12) having pivot centres (11, 12) with vertical axles in the region of each side edge of the table (1) enabling the receptor unit to be swung out to alternate positions on either side of the table (1) and therefrom swung up to a vertical position about a horizontal axle (10), in which vertical positions it is parallel with the table for operating with horizontal beam paths in mutually opposite directions.

2. A table according to Claim 1, **characterized in that** the articulated structure includes a pivot axle(25) which in a outwardly and up- or downwardly swung receptor position is vertical and permits operation with an angled beam path.

3. A table according to Claim 1, **characterized in that** the articulated structure comprises arms (7, 9) which are joined together by a link (8) via said pivot centres (11, 12) having vertical axles in the region of each side edge of the table (1), and a horizontal hinge (10) which connects the unit to one (9) of said arms.

4. A table according to Claim 3, **characterized in that** the receptor unit (2) can be swung from the position for operation with a horizontal beam path to a position perpendicular to the table, through the medium of a vertical axle.

5. A table according to Claim 4, **characterized in that** the vertical axle constitutes one (11) of said pivot centres (11, 12).

6. A table according to Claim 3, **characterized in that** the lengths of the arms (7, 9) and the link (8) are such that the receptor unit (2) will take a position for operation with a centered beam path without being moved in the X-direction from its original position (position A) in or beneath the table (1), irrespective of from which side the receptor unit is swung outwards and upwards.

7. A table according to Claim 3 or 4, **characterized in that** the receptor unit (1) and its associated arms (7, 9), link (8) and pivot centres (11, 12) are supported by a carriage mounted in or on the underside of the table (1) and movable in the X-direction.

8. A table according to any one of Claims 1-7,
**characterized in that** the table top is movable in the X-direction and/or the Y-direction, and **in that** the receptor unit (1) is mounted in a frame carried by said table top.

9. A table according to Claim 7 or Claim 8, **characterized in that** the carriage or the frame includes an element (15) having means (16-19) for guiding movement of a further element (18) journalled to one (7) of the arms (7, 9), wherein the other (9) of said arms carries a block (20) in which a plate (24) carrying the receptor unit (20) is journalled for pivotal movement about a horizontal axle (10).

10. A table according to Claim 9, **characterized in that** said block (20) has a further axle (21) which is parallel with said horizontal axle (10) and on which said plate (24) supporting said receptor unit is pivotally mounted to take a vertical position below the table (1). (Fig. 17).

11. A table according to Claim 10, **characterized in that** the receptor unit (2) is connected to the plate (24) by means of an axle (25) which extends perpendicularly to the plate and about which the receptor unit can be swung for operation with an angled beam path.

12. A table according to any one of Claims 1-11, **characterized in that** the table, its frame, the carriage or the receptor unit includes an operating device, e.g. a joy-stick, for manoeuvering the linear movement and pivotal movement of the receptor unit (2) in relation to the patient support table.

## Patentansprüche

1. Ein Patientenlagerungstisch, ausgestattet mit einer Empfängereinheit und gedacht zur Strahlungserfassung wie zum Beispiel Röntgenstrahlphotographie oder elektronische Bildspeicherung, wobei die Empfängereinheit (2) für die Bewegung in eine X-Richtung parallel zu der longitudinalen Richtung des Tisches (1) gelagert ist;
geeignet für das Zusammenwirken mit einer Strahlungsquelle, welche verschiebbar ist in der X-Richtung, einer Y-Richtung parallel zu der querverlaufenden Richtung des Tisches und in einer Z-Richtung;
geeignet, um um eine horizontale Achse geschwenkt zu werden; und ausschwenkbar und aufschwenkbar um eine vertikale und eine horizontale Achse von einer Position in oder unterhalb einer oberen Oberfläche des Tisches (1) im Falle eines vertikalen Strahlenpfades zu einer Position auf einer Seite des Tisches und parallel zum Tisch im Falle eines horizontalen Strahlenpfades,
**dadurch gekennzeichnet,**
**dass** die Empfängereinheit (2) durch eine Gelenkkonstruktion (7 bis 12) gelagert ist, die Drehzentren (11, 12) mit vertikalen Achsen im Bereich von jeder Seitenkante des Tisches (1) aufweist, die es der Empfängereinheit ermöglichen, zu wechselseitigen Positionen auf beiden Seiten des Tisches (1) ausgeschwenkt zu werden, und von da aus zu einer vertikalen Position um eine horizontale Achse (10) aufgeschwenkt zu werden, in welchen vertikalen Positionen sie parallel zum Tisch verläuft, um mit horizontalen Strahlenpfaden in wechselseitig entgegengesetzten Richtungen betätigt zu werden.

2. Tisch nach Anspruch 1, **dadurch gekennzeichnet, dass** die Gelenkkonstruktion eine Drehachse (25) umfasst, die in einer nach außen und auf- oder abwärts geschwungenen Empfängerposition vertikal verläuft und den Betrieb mit einem angewinkelten Strahlenpfad gestattet.

3. Tisch nach Anspruch 1, **dadurch gekennzeichnet, dass** die Gelenkkonstruktion Träger (7, 9) umfasst, die durch ein Verbindungsstück (8) über Drehzentren (11, 12) zusammengefügt werden, welche vertikale Achsen im Bereich von jeder Seitenkante des Tisches (1) aufweisen, und ein horizontales Scharnier (10), welches die Einheit mit einem (9) der Träger verbindet.

4. Tisch nach Anspruch 3, **dadurch gekennzeichnet, dass** die Empfängereinheit (2) durch eine vertikale Achse von der Betriebsposition mit einem horizontalen Strahlenpfad zu einer Position rechtwinklig zum Tisch geschwungen werden kann.

5. Tisch nach Anspruch 4, **dadurch gekennzeichnet, dass** die vertikale Achse eine (11) der Drehzentren (11, 12) bildet.

6. Tisch nach Anspruch 3, **dadurch gekennzeichnet, dass** die Längen der Träger (7, 9) und das Verbindungsstück (8) derart gestaltet sind, dass die Empfängereinheit (2) eine Betriebsposition mit einem zentrischen Strahlenpfad einnimmt, ohne von ihrer Ausgangsposition (Position A) in oder unterhalb des Tisches (1) in die X-Richtung verschoben zu werden, ungeachtet von welcher Seite die Empfängereinheit auswärts und aufwärts geschwungen wird.

7. Tisch nach Anspruch 3 oder 4, **dadurch gekennzeichnet, dass** die Empfängereinheit (2) und ihre verbundenen Träger (7, 9), Verbindungsstück (8) und Drehzentren (11, 12) durch einen Schlitten unterstützt werden, welcher in oder auf der Unterseite des Tisches montiert und in der X-Richtung verschiebbar ist.

8. Tisch nach einem der Ansprüche 1 bis 7, **dadurch gekennzeichnet, dass** das Tischoberteil in der X-Richtung und/oder der Y-Richtung verschiebbar ist und in welchem die Empfängereinheit (2) in einem Rahmen montiert ist, der durch das Tischoberteil getragen wird.

9. Tisch nach Anspruch 7 oder 8, **dadurch gekennzeichnet, dass** der Schlitten oder der Rahmen ein Element (15) umfasst, welches Mittel (16-19) zum Steuern der Bewegung eines weiteren Elementes (18) aufweist, das an einen (7) der Träger (7, 9) gelenkig gelagert ist, wohingegen der andere (9) der Träger einen Block (20) trägt, in welchen eine Platte (24), die die Empfängereinheit (2) trägt, zur Drehbewegung um eine horizontale Achse (10) gelenkig gelagert ist.

10. Tisch nach Anspruch 9, **dadurch gekennzeichnet, dass** der Block (20) eine weitere Achse (21) hat, welche parallel zur horizontalen Achse (10) ist, und auf welchem die Platte (24), die die Empfängereinheit trägt, drehbar montiert ist, um eine vertikale Position unter dem Tisch (1) einzunehmen. (Figur 17).

11. Tisch nach Anspruch 10, **dadurch gekennzeichnet, dass** die Empfängereinheit (2) mit der Platte (24) mittels einer Achse (25) verbunden ist, welche sich rechtwinklig zu der Platte erstreckt und um welche die Empfängereinheit (2) für den Betrieb mit einem angewinkelten Strahlenpfad geschwungen werden kann.

12. Tisch nach einem der Ansprüche 1 bis 11, **dadurch gekennzeichnet, dass** der Tisch, sein Rahmen, der Schlitten oder die Empfängereinheit eine Betätigungsvorrichtung umfasst, zum Beispiel einen Joy-Stick, um die lineare Bewegung und die Drehbewegung der Empfängereinheit (2) in bezug auf den Patientenlagerungstisch zu manövrieren.

## Revendications

1. Table support pour patient munie d'une unité de réception et destinée à la détection de rayonnement tel qu'une radiographie ou à la mémorisation d'une image électronique, dans laquelle l'unité de réception (2) :
est supportée, pour se déplacer, dans une direction X parallèle à la direction longitudinale de la table (1);
est conçue pour une coaction avec une source de faisceau qui peut se déplacer dans la direction X, une direction Y parallèle à la direction transversale de la table, et une direction Z ;
est conçue pour basculer autour d'un axe horizontal ; et
est capable de basculer vers l'extérieur et vers le haut autour d'un axe horizontal et d'un axe, d'une position située dans ou sous une surface supérieure de la table (1), dans le cas d'un chemin de faisceau vertical, jusqu'à une position située sur un côté de, et parallèle à la table, dans le cas d'un chemin de faisceau horizontal,
**caractérisée en ce que**
l'unité de réception (2) est supportée par une structure articulée (7 - 12) ayant des centres de pivot (11, 12) munis d'axes verticaux dans la région de chaque bord latéral de la table (1) permettant à l'unité de réception de basculer vers l'extérieur jusque dans des positions alternatives, de chaque côté de la table (1) et, depuis celles-ci, de basculer vers le haut jusqu'à une position verticale autour d'un axe horizontal (10), dans lesquelles positions verticales elle se trouve parallèle à la table afin de fonctionner avec des chemins de faisceau horizontaux dans des directions opposées mutuellement.

2. Table selon la revendication 1, **caractérisée en ce que** la structure articulée comporte un axe de pivot (25) qui, dans une position de récepteur basculée vers l'extérieur et vers le haut, ou vers le bas, est verticale et permet le fonctionnement avec un chemin de faisceau incliné.

3. Table selon la revendication 1, **caractérisée en ce que** la structure articulée comprend des bras (7, 9) qui sont réunis ensemble grâce à une liaison (8), à travers les centres de pivot (11, 12) ayant des axes verticaux dans la région de chaque bord latéral de la table (1), et une charnière horizontale (10) qui raccorde l'unité à l'un (9) desdits bras.

4. Table selon la revendication 3, **caractérisée en ce que** l'unité de réception (2) peut basculer de la position de fonctionnement avec un chemin de faisceau horizontal jusqu'à une position perpendiculaire à la table, grâce à un axe vertical.

5. Table selon la revendication 4, **caractérisée en ce que** l'axe vertical constitue l'une (11) desdits centres de pivot (11, 12).

6. Table selon la revendication 3, **caractérisée en ce que** les longueurs des bras (7, 9) et de la liaison (8) sont telles que l'unité de réception (2) prend une position de fonctionnement avec un chemin de faisceau centré sans être déplacée dans la direction X depuis sa position originale (position A) située dans ou sous la table (1), quel que soit le côté de l'unité de réception depuis lequel l'unité de réception bascule vers l'extérieur et vers le haut.

7. Table selon la revendication 3 ou 4, **caractérisée en ce que** l'unité de réception (2) et ses bras associés (7, 9), la liaison (8) et les centres de pivot (11, 12) sont supportés par un chariot monté dans ou sur le dessous de la table (1) et peuvent se déplacer dans la direction X.

8. Table selon l'une quelconque des revendications 1 à 7, **caractérisée en ce que** le dessus de la table peut se déplacer dans la direction X et/ou dans la direction Y, et **en ce que** l'unité de réception (2) est montée dans un cadre porté par ledit dessus de table.

9. Table selon la revendication 7 ou 8, **caractérisée en ce que** le chariot ou le cadre comporte un élément (15) ayant des moyens (16 - 19) permettant de guider le mouvement d'un autre élément (18) tourillonnant, sur l'un (7) des bras (7, 9), dans laquelle l'autre desdits bras porte un bloc (20) dans lequel une plaque (24) portant l'unité de réception (2) tourillonne, pour permettre un mouvement pivotant autour d'un axe horizontal (10).

10. Table selon la revendication 9, **caractérisée en ce que** ledit bloc (20) a un autre axe (21) qui est parallèle au dit axe horizontal (10) et sur lequel ladite plaque (24) supportant ladite unité de réception est montée, pour pivoter, afin de prendre une position verticale sous la table (1). (Figure 17).

11. Table selon la revendication 10, **caractérisée en ce que** l'unité de réception (2) est raccordée à la plaque (24) au moyen d'un axe (25) qui s'étend perpendiculairement à la plaque et autour duquel l'unité de réception peut basculer pour fonctionner avec un chemin de faisceaux angulaire.

12. Table selon l'une quelconque des revendications 1 à 11, **caractérisée en ce que** la table, son cadre, le chariot ou l'unité de réception comporte un dispositif de fonctionnement, par exemple, un manche de commande, permettant d'exécuter le mouvement linéaire et le mouvement pivotant de l'unité de réception (2) par rapport à la table de support de patient.
